(19) Europäisches Patentamt
European Patent Office
Office européen des brevets

(11) **EP 1 634 532 B1**

(12) **EUROPEAN PATENT SPECIFICATION**

(45) Date of publication and mention
of the grant of the patent:
**03.06.2009  Bulletin 2009/23**

(51) Int Cl.:
***A61B 5/103*** (2006.01)

(21) Application number: **05255513.3**

(22) Date of filing: **08.09.2005**

(54) **Method of measuring skin anisotropy**

Methode zur Messung von Haut Anisotropie

Méthode de mesure de l'anisotropie de la peau.

(84) Designated Contracting States:
**DE FR GB IT**

(30) Priority:  **09.09.2004  US 937039**

(43) Date of publication of application:
**15.03.2006  Bulletin 2006/11**

(73) Proprietor: **JOHNSON & JOHNSON CONSUMER COMPANIES, INC.**
**Skillman,**
**New Jersey 08558 (US)**

(72) Inventors:
• **Ruvolo, Eduardo**
**Plainsboro, NJ 08536 (US)**
• **Kollias, Nikiforos**
**Skillman, NJ 08558 (US)**
• **Cole, Curtis**
**Ringoes, NJ 08551 (US)**

(74) Representative: **Fisher, Adrian John et al**
**Carpmaels & Ransford**
**43-45 Bloomsbury Square**
**London WC1A 2RA (GB)**

(56) References cited:
**US-A- 5 115 808          US-A1- 2002 029 924**
**US-B1- 6 168 572**

• **REIHSNER R ET AL: "TWO-DIMENSIONAL ELASTIC PROPERTIES OF HUMAN SKIN IN TERMS OF AN INCREMENTAL MODEL AT THE IN VIVO CONFIGURATION" MEDICAL ENGINEERING & PHYSICS, BUTTERWORTH-HEINEMANN, GB, vol. 17, no. 4, June 1995 (1995-06), pages 304-313, XP000775783 ISSN: 1350-4533**

**Description**

BACKGROUND OF THE INVENTION

The skin is a sensory organ, an immune organ, an organ that provides thermoregulation, and a barrier to chemical and biophysical species acting in both directions inwards and outwards. The skin is also a structural organ that interacts with other structural elements such as bones and muscles. The bones are the body's structural elements onto which tissue elements are attached. Muscles surround the bones, and tendons provide attachments between the bones and the muscles allowing forces to be applied onto the bones, which may result in motion or locomotion. The muscles are surrounded by layers of fat that allow changes in volume of the muscles as they flex while maintaining a relatively constant outline. Muscles also serve as insulation and act as shock absorbers to external impulses. The skin is the wrapping that keeps the subcutaneous fat in position and defines in space the body from external elements. The skin, thus, is an element that is generally under tension, allowing flexing and locomotion by adjusting and redistributing internally, as well as externally, applied forces.

The mechanical properties of the skin are of paramount importance in describing the state of the skin as a container of the body, both as a barrier and an enclosure. A number of techniques have been developed to study the mechanical properties of the skin. These techniques are based on the idea that assessing the force necessary to pull or to push the skin allows one to estimate the elastic and plastic properties of the skin since the skin undergoes both elastic and plastic deformations.

It is evident to any observer that the state of the skin changes significantly with age. In particular, it is known that skin generally loses elasticity as it ages. This is attributed to skin thinning and loss of elastin and collagen in the dermal matrix, as well as losses in the subcutaneous tissue (fat layers and muscle mass), which are expressed as sagging of the skin. The mechanical properties of the skin are, in particular, heavily influenced by the microstructural arrangement of collagen and elastin in the dermal matrix. Collagen forms fibrils that appear as fibers and bundles that are believed to be arranged in a chicken wire fence pattern, allowing the dermis to deform due to pressure and, thus, minimizing the possibility of tears. The collagen bundles vary in size as one moves from the upper (papillary) dermis to the deeper (reticular) dermis and are normally under tension that ranges from 0-20 N/m depending on the body site, direction, and posture (Y. Lanir, Skin Mechanics-in: Handbook of Bioengineering eds. Richard Skalak and Shu Chien,. MacGraw-Hill Book Co chapter 11 pp 11.1-11.24.).

Collagen production takes place preferentially along the direction of tension of the fibroblasts (the dermal cells that are responsible for the production of collagen). The other structural dermal element is elastin, which appears as bundles and is interspersed in the collagen matrix. Elastin bundles form a two dimensional network within the dermal collagen, and the bundles also reach towards the dermal epidermal junction forming candelabra-like structures.

Such observations regarding the arrangement of collagen and elastin in the dermis, as well the ability of this arrangement to change with age, is consistent with the understanding that tension the skin is under is directional. This has, in fact, been used to advantage in surgical procedures. For example, maximum tension in the skin has been found to orient along Langer's cleavage lines present in the skin. As such, this orientation is typically chosen as the direction along which surgical incisions are made so that the tension across the wound is minimized.

While various instrumental approaches have been described for objectively assessing the elastic or more correctly the mechanical properties of the skin, these approaches have frequently failed to account for anisotropy in mechanical parameters of the skin. One such method employs an instrument that generates suction. The height to which the skin may be pulled under constant suction is determined, and then the rate at which the skin returns to its original shape is also measured. Another method uses two concentric cylinders that are placed in contact with the skin. One of the cylinders applies a constant torque to the skin surface and measures the angular displacement under torque and the rate at which the skin returns to equilibrium once the torque is removed. Yet another method uses an instrument where a small mass located on an arm is allowed to strike the skin with a certain fixed velocity and it determines the speed and the rebound of the mass from the skin thereby assessing the firmness/elasticity of the skin. Unfortunately, while these measurement methods can successfully measure certain elastic properties of the skin, they do not take into consideration the anisotropy that is provided by the arrangement of dermal collagen and elastin. As such, these methods have limitations in both poor sensitivity/resolution and random testing error. These problems make the above methods unreliable and unsatisfying, especially for determining fine or even gross effects from treating the skin with skin care compositions or other treatments.

One tool that attempts to overcome the problems of directional insensitivity of the instruments and methods described above is the Reviscometer® RVM 600 (commercially available from Courage and Khazaka, Cologne, Germany). The instrument measures the time of propagation of an elastic shear pulse in viscoelastic materials such as skin. The principle behind the use of the instrument is that the speed of propagation of elastic disturbances on the skin will depend strongly on its orientation because it depends on both the tension the tissue is under and the density of the tissue. Mechanical vibrations propagate faster the higher the tension. As with a guitar string, the higher the tension, the higher the frequency of oscillation after plucking. A probe that is placed in contact with the skin is composed of two transducers that are spaced apart and mounted on two independent supports. One transducer generates a motion of small amplitude and the second transducer determines when the disturbance generated by the first transducer arrives at its location.

The manufacturer of the Reviscometer in its operations manual recommends measurements at 45° increments in order to assess the variability of skin firmness with skin direction. Unfortunately, this method yields highly inconsistent measurements, and is unable to provide a high degree of resolution with respect to subtle differences in firmness. Thus, it is very difficult to, for example, differentiate the measurement of the firming effect of active topical products versus a placebo control product. Accordingly, there remains a need to overcome the above-mentioned drawbacks.

US 6,168,572, US 2002/0029924 and US 5,115,808 each disclose methods of determining skin anisotropy of a subject of the type set forth in the preamble of the accompanying claim 1.

## SUMMARY OF THE INVENTION

In one aspect, as defined in the accompanying claim 1, the present invention features a method of determining skin anisotropy of a subject by measuring rates of propagation of mechanical energy between a mechanical energy generator and a mechanical energy detector along a plurality of directions of an expanse of skin wherein each of the directions are from about 0° to about 10° in separation relative at least one other of the directions and at least two of the directions are from about 30° to about 180° in separation relative to each other.

In another aspect of the invention as defined in the accompanying claim 11, the present invention features a method of determining the efficacy of a skin treatment that includes the steps of: (i) determining a first skin anisotropy measurement of a subject by the above-described method of claim 1,(ii) administering a non-surgical cosmetic treatment to the expanse of skin; (iii) determining a second skin anisotropy measurement of the subject by the above-described method of claim 1;and (iv) comparing the first and the second skin anisotropy measurements.

Other features and advantages of the present invention will be apparent from the detailed description of the invention and from the claims.

## BRIEF DESCRIPTION OF THE DRAWINGS

A more particular description of the invention, briefly summarized above may be had by reference to the embodiments thereof that are illustrated in the appended drawings. It is to be so noted, however, that the appended drawings illustrate only typical embodiments of the invention and, therefore, are not to be considered limiting of its scope, for the invention may admit to other equally effective embodiments.

Figure 1 is a top perspective view of a skin measurement device that may be used to practice embodiments of the invention described herein.

Figure 2A is a top perspective view of an expanse of skin having transducers of the skin measurement device of Figure 1 placed thereon, in order to measure a first rate of propagation of mechanical energy, according to embodiments of the invention described herein.

Figure 2B is a top perspective view of the expanse of skin of Figure 2A, wherein the transducers have been moved to measure a second rate of propagation of mechanical energy, according to embodiments of the invention described herein.

Figure 3 is a side perspective view of a probe head being placed within a holding ring, according to embodiments of the invention described herein.

Figure 4 is a plot of resonance running time versus probe angle and a mathematical model fit of such data measured for a particular expanse of skin.

Figure 5 is a plot of resonance running time versus probe angle for three separate individual subjects, each falling within a different age group.

Figure 6 is a plot of two different skin anisotropy parameters and their variation with age of the subjects.

Figure 7 is a plot of a skin anisotropy parameter versus subject age and a mathematical model fit for such data.

Figure 8 is a correlation between actual age of subjects that underwent resonance running time measurements and their age as calculated using a mathematical model.

Figure 9 is a plot of a skin anisotropy parameter versus probe angle for measurements performed upon the neck area of various subjects.

Figure 10 is a plot of resonance running time versus probe angle for a particular expanse of skin, further depicting the loss of information if one limits the measurements to large angle separations.

Figure 11 is a plot of resonance running time versus probe angle for a particular expanse of skin before and after the expanse of skin is treated with the benefit agent DMAE.

Figure 12 is a plot showing the degree to which a skin anisotropy parameter is enhanced from treatment of the skin with compositions containing various levels of the benefit agent DMAE.

To facilitate understanding identical reference elements have been used, wherever possible, to designate identical elements that are common to the figures.

DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs.

Skin Anisotropy

Embodiments of the present invention relate to a method of determining skin anisotropy. What is meant by "skin anisotropy" is the magnitude or degree to which at least one property of the skin varies depending upon which direction (relative to an arbitrary direction) along which the property is measured. For example, if an arbitrary direction along the skin is defined as 0°, a measurement of a rate of propagation of a mechanical (e.g., sound) wave along 0° and a measurement along an angle other than 0° are taken. If the measurements are significantly different, the skin is said to be anisotropic with respect to sound propagation. The degree to which the measurements differ is a determination of skin anisotropy. By "rate of propagation" it is meant a speed at which a wave or pulse of mechanical energy moves across an expanse of skin, such as a rate at which the mechanical energy moves between two transducers. According to embodiments of the invention described herein, skin anisotropy may be determined by relating, for example, a first rate of propagation of mechanical energy along a first direction of skin to a second rate of propagation of mechanical energy of skin along a second direction of skin. By "relating a first rate of propagation of mechanical energy along a first direction of skin to a second rate of propagation of mechanical energy along a second direction of skin," it is meant that a graphical or mathematical relationship between such rates is determined.

Measurement Device

According to embodiments of the invention described herein, skin anisotropy of a subject is measured by measuring rates of propagation of mechanical energy along a plurality of discrete directions of an expanse of skin. In one embodiment, the subject is a mammal such as a human. The method of the present invention may be used on both healthy subjects (e.g., to ensure their skin health) as well as subjects who are inflicted at various stages of a skin disorder, including but not limited to intrinsic skin aging, wrinkles, crow's feet, photodamage, swelling (edema), and the like.

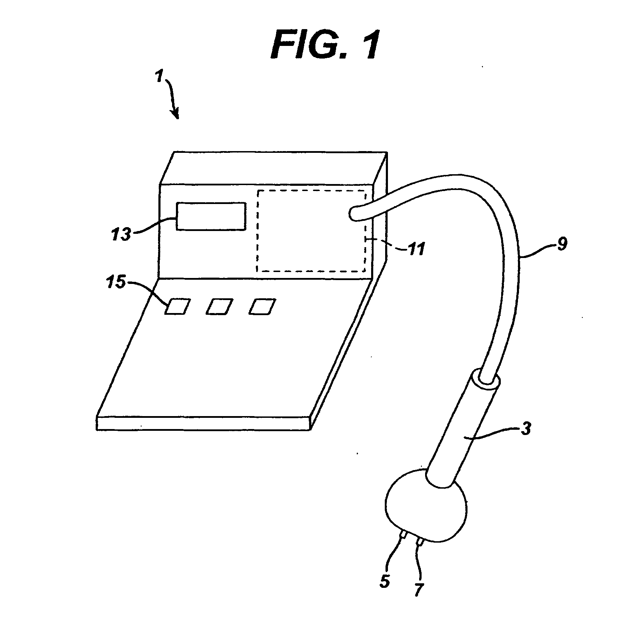

A suitable device for measuring rates of propagation of mechanical energy is the Reviscometer® RVM 600 (commercially available from Courage and Khazaka, Cologne, Germany), which is depicted in Figure 1. Referring to Figure 1, a device 1 for measuring the propagation of mechanical energy includes a probe unit 3 having a mechanical energy generator, such as a first transducer 5, for transmitting mechanical energy and a mechanical energy detector, such as a second transducer 7. In one embodiment, transducer 5 and transducer 7 are spaced apart by a distance of from about 1.5 to about 2 mm. The transducers may be mounted on two independent supports with pressure sensors (not shown) coupled thereto, in order to ensure proper contacting force prior to the generation or detecting of the mechanical energy.

The transducer 5 and transducer 7 are electrically coupled, via a connector 9, to a signal unit 11 (shown in phantom in Figure 1). The signal unit 11 generally includes a power supply and various sub-elements for the generation, analysis, processing and control of signals that are generated and processed by the device. Such sub-components (not shown in Figure 1) include, for example, a pulse generator for generating pulses of mechanical energy, an amplifier for amplifying signals, and microprocessor for controlling signals, as well as other components for signal analysis. An example of a suitable block diagram of various sub-components that may be included in the signal unitis set forth in "Evaluation of Skin Viscoelasticity and Anisotropy by Measurement of Speed of Shear Wave Propagation With Viscoelasticity Skin Analyzer," (Vexler et al; The Journal of Investigative Dermatology, Inc., Vol. 115,no 5, pp 732-739, 1999). Device 1 further includes a display 13 that is coupled to the signal unit 11 for viewing output generated by the device 1, as well one or more controls 15 for sending an electrical pulse that is converted into mechanical pulse by transducer 5.

Note, that while the device described above includes transducer 5 for converting electrical energy to mechanical energy (to generate the energy to be propagated across the expanse of skin) and transducer 7 for converting mechanical energy back to electrical energy (for signal processing), other types of devices, such as those that may be fabricated on integrated circuits may be used. For example, transducer 5 may be a pulsed laser that uses electromagnetic radiation to generate a mechanical wave that is in turn propagated across the skin. Corresponding transducer 7 may include a photo-acoustic or electro-acoustic material such as lead zirconate titanate (PZT) that is capable of converting the mechanical energy that has propagated across the skin into an electrical or optical signal to be processed. In this embodiment, transducer 7 may be a plurality of photo-acoustic detectors each spaced apart, such as in a circular manner at a constant distance from transducer 5 at a particular angle (e.g., 0° , 3°, 6°, 9°, and the like) relative to transducer 5. Each of the plurality of photo-acoustic detectors captures a signal that can be correlated to a rate of propagation of mechanical energy across the expanse of skin 17 in a particular direction. Such a configuration of transducer 5 and transducers 7 may be fabricated, for example, on an integrated circuit using techniques known in the art of integrated circuit manufacture.

<u>Method of Measuring Rate of Propagation of Mechanical Energy Along an Expanse of Skin</u>

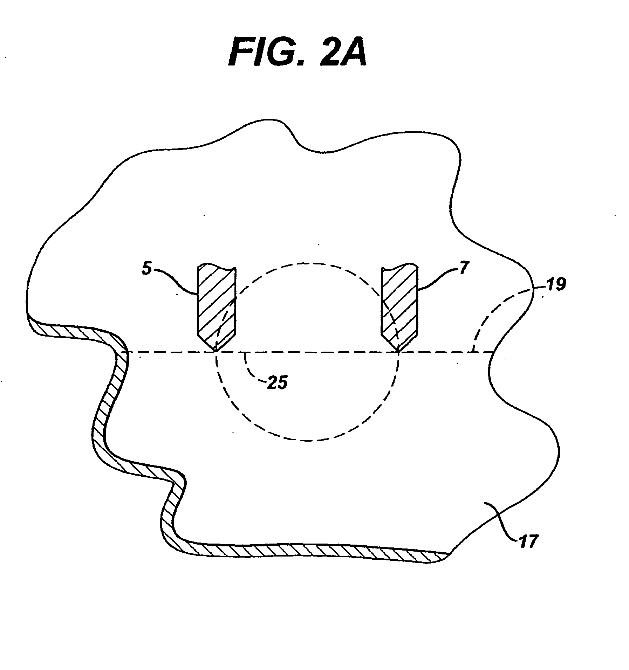

In operation, the transducer 5 and transducer 7 are placed in contact with an expanse of skin 17 along a first arbitrary direction 19 as shown in Figure 2A and as described in the operations manual for the Reviscometer® RVM 600. The expanse of skin may be cleansed before taking measurements, but this is not required. The controls 15 are depressed or selected in order to begin the propagation of mechanical energy (e.g., an acoustic wave such as an elastic shear wave) from the first transducer 5.

The mechanical energy may, for example, be in the form of a pulse that propagates from the first transducer 5 across the expanse of skin 17 to the second transducer 7 along a first segment of propagation 25. In one embodiment, the pulse has a frequency in a range from about 0.5kHz to about 30kHz. The microprocessor then calculates one or more parameters of the propagation (e.g., that can later be correlated to the density or firmness of the expanse of skin 17). For example, the microprocessor may calculate a time required for the pulse to move from the first transducer 5 across the expanse of skin 17 along first arbitrary direction 19 to the second transducer 7. The time required is referred to as a resonance running time (RRT). Similarly, by factoring in the distance between transducer 5 and transducer 7, the microprocessor may calculate a velocity of propagation of the pulse.

By spacing transducer 5 and transducer 7 apart at a distance from about 1.5 to about 2 mm, the instrument probes the propagation of mechanical energy through the epidermis and superficial dermis. It, however, is believed that useful measurements may also be obtained using a spacing as small as about 0.5 mm or as large as about 5mm.

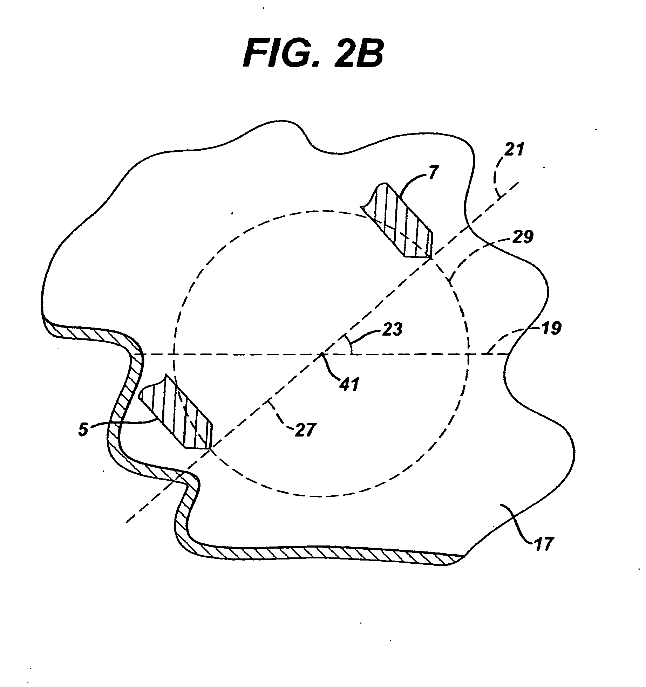

As shown in Figure 2B, a second rate of propagation is then measured along a second direction 21 that is displaced from the first direction 19 by a probe angle 23. In one embodiment, the probe angle 23 is between about 0° and about 10° (in other words, the second rate of propagation is measured along a direction that is within about 10° in separation from the direction along which the first rate of propagation is measured), such as between about 0° and about 5° (e.g., less than 5 °), such as between about 0° and about 3° (e.g., less than 3°). In one embodiment of the invention, the measurement of the second rate of propagation includes moving the transducer 5 and transducer 7 along the expanse of skin such that they are separated by a second segment of propagation 27. The first segment of propagation 25 and the second segment of propagation 27 may be co-centric, as shown in Figure 2B (i.e., the transducer 5 and transducer 7 are rotated such that they remain on a boundary of an imaginary circle 29 (shown in phantom in Figure 2B). In this embodiment of the invention, the first segment of propagation and the second segment of propagation intersect at a centerpoint 41.

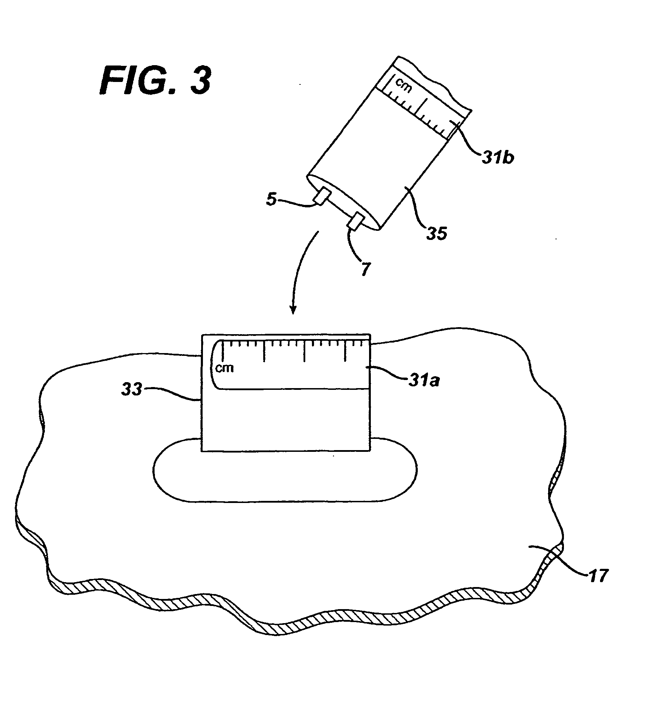

Referring to Figure 3, in order to facilitate determining or setting of probe angle 23, the probe unit 3 may include a separate holding ring 33 having a hollow interior through which a probe head 35 may be inserted (the holding ring 33 and its function are described in the supplier literature for the Reviscometer® RVM 600). In this embodiment of the invention, the holding ring 33 is attached to the expanse of skin 17 via, for example, double sided tape, to fix the holding ring thereto. The probe head 35 is inserted through the hollow interior of the holding ring 33 such that the transducers 5, 7 contact the expanse of skin 17. In order to make accurate measurements of the probe angle 23, a ruler 31 a may be first attached to the holding ring 33 and a mating ruler 31b to the probe head 35. The ruler 31a and the ruler 31b may be aligned to an arbitrary angle of 0, and the first measurement is then taken. The probe head 35 is then rotated with respect to the holding ring 33 a number of millimeters on the ruler 31a and ruler 31 B that correspond to the desired probe angle 23 (e.g., 1mm corresponding to about 3°). Note that the relationship between the number centimeters that the probe head 35 is rotated is related to the angle by the diameter of the holding ring 33 (e.g., the angle is equal to 360° times the length of rotation in centimeters divided by the product of p times the diameter of the holding ring 33).

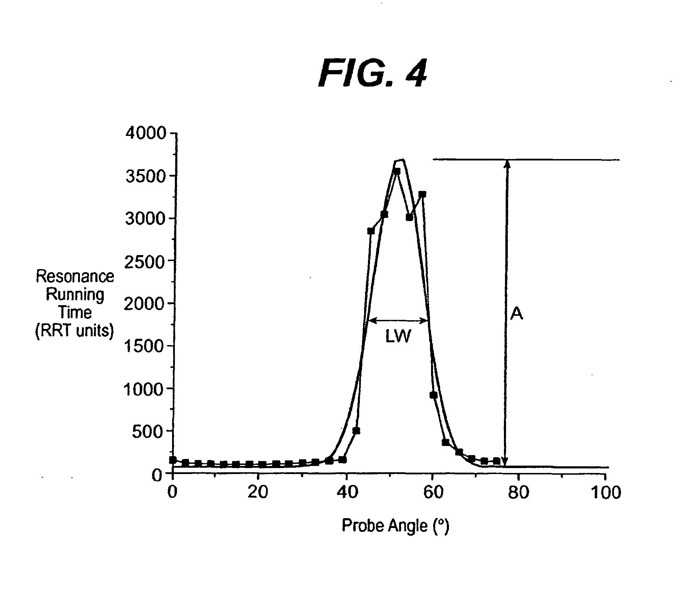

Once the probe head is adjusted, the second measurement is taken. The steps of rotating the probe head 35 and taking an additional measurement is repeated one or more times, such as, for example, to cover span of angles up to at least 30°, but as much as 90°, 120°, or even 180° from the first (arbitrary) direction 19. In general, rates of propagation of mechanical energy are measured between transducer 5 and transducer 7, along a plurality of directions of the expanse of skin 17. Each of the directions are from about 0° to about 10° in separation relative at least one other of the directions, and at least two of the directions are at least about 30° in separation relative to each other. In this manner, one is able to obtain enhanced resolution of anisotropy. See, e.g., Figures 4, 5, 10, and 11 and the description in the text under "EXAMPLES." Note that for measurement convenience, one may take readings in both "senses" from the arbitrary direction 19 (e.g, clockwise from direction 19: 5°, 10°, and 15° and then counterclockwise from direction 19: 5 °, 10 °, and 15°) in order to resolve anisotropy with a minimal amount of measurements.

Note that for embodiments of the invention in which transducer 5 converts optical energy from a pulse of light into mechanical energy which is propagated across the expanse of skin 17, and transducer 7 is a plurality of photo-acoustic detectors each spaced apart in a circular manner (described above in the section, "MEASUREMENT DEVICE"), measurements at various angles are conveniently measured in a simultaneous manner (e.g., no rotation of transducers is required).

**Assessing Skin Anisotropy and Calculating Skin Anisotropy Parameters**

Skin anisotropy may be assessed by relating the first rate of propagation of mechanical energy along the first direction of the expanse of skin to the second rate of propagation of mechanical energy along the second direction of the expanse of skin. This may be accomplished by plotting resonance running time versus probe angle. In one embodiment of the invention, relating the first rate of propagation of mechanical energy to the second rate of propagation includes calculating a skin anisotropy parameter. The skin anisotropy parameter is generally calculated from the at least two measurements of rates of propagation of mechanical energy. In particular, the anisotropy parameter may be derived from or include a difference, a quotient, or a ratio between (1) the time or velocity of propagation determined by the first measurement and (2) the time or velocity of propagation determined by the second measurement. For example, if time of propagation is measured, the anisotropy parameter may be derived from a ratio of the first time of propagation to the second time of propagation. If numerous measurements are taken, the relationship between time of propagation versus angle may be modeled as a Gaussian or other suitable mathematical function to determine a maximum and minimum time of propagation. A ratio between maximum and minimum RRT may then be used as the anisotropy parameter. Specific examples of how anisotropy parameters may be calculated are discussed below in the section entitled "EXAMPLES."

The Expanse of Skin

Various locations may be chosen for the expanse of skin 17. In one embodiment of the invention, the expanse of skin is relatively loose and fleshy such as skin located on the upper inner arm, the neck, upper inner thigh, the abdomen, buttocks, or other soft body parts (e.g., where any bone is well buried beneath soft tissue). The skin on the upper inner arm is particularly preferred. In another embodiment of the invention, the expanse of skin is located in a region that is not prone to a high degree of exposure to the sun, such as the upper inner arm or the buttocks.

Skin Treatments

What is meant by a "skin treatment" is a non-surgical cosmetic treatment of the expanse of skin with a therapeutic device (e.g., mechanical, optical, or electrical device) or a benefit agent (e.g., delivered via such routes as topical or oral compositions) that may effect the skin's elasticity, density, firmness, number or frequency of wrinkles, or other indications of aging. "Applying a skin treatment" refers administering the therapeutic device to the expanse of skin (e.g., contacting the expanse of skin with a mechanical device or illuminating it with a light source) or applying a benefit agent (e.g., such as by topically applying a composition containing the benefit agent to the expanse of skin). What is meant by a "benefit agent" is a compound (e.g., a synthetic compound or a compound isolated from a natural source) that has a cosmetic or therapeutic effect on the skin, including, but not limiting to, anti-aging agents, firming agents, and anti-wrinkle agents. Examples of benefit agents include, but are not limited to, vitamin A and its derivatives such as beta-carotene and retinoids such as retinoic acid, retinal, retinyl esters such as and retinyl palmitate, retinyl acetate, and retinyl propionate;

vitamin C and its derivatives such as ascorbic acid, ascorbyl phosphates, ascorbyl palmitate and ascorbyl glucoside; copper peptides; simple sugars such as lactose, mellibiose and fructose; and alkanolamines such as dimethylaminoethanol ("DMAE")

Evaluation and Promotion of Skin Treatments

Skin treatments may be evaluated, advertised, or promoted in conjunction with embodiments of the inventive method for determining skin anisotropy described herein. For example, a plurality of pre-treatment rates of propagation of mechanical energy may be measured for an expanse of skin of a subject using a device such as device 1. After this measurement, a skin treatment may be applied the expanse of skin of the subject. After the application of the skin treatment, post-treatment measurements may be made. One or more of the pre-treatment rates of propagation of mechanical energy may then be compared to one or more of the post-treatment rates of propagation of mechanical energy to evaluate the efficacy of the skin care treatment.

In another embodiment of the invention, a relationship is determined between skin anisotropy parameters and other variables such as chronological skin age. The relationship may be determined by, for example, making skin anisotropy measurements of a plurality of subjects who are classified in one or more individual categories such as categories based upon age, sex, ethnicity, skin type, skin condition, or combinations of these categories. An anisotropy parameter associated with a test subject is then determined. The anisotropy parameter of the test subject is then compared to one or more standard anisotropy parameters determined above (e.g., to classify the subject into or compare the subject with a particular age group). A skin treatment may then be applied to the subject and then a post-treatment anisotropy parameter may be determined. The post-treatment anisotropy parameter may then be compared with the standard anisotropy parameter and/or with the pre-treatment anisotropy parameter to classify the subject into or compare the subject with a group or to measure the degree of improvement after the skin treatment.

A method of promoting the use of a product, such as a topical composition or skin treatment, may include promoting the use of the product for reducing the appearance of the age of a user's skin, wherein the efficacy of the product was determined using by measuring skin anisotropy in a manner consistent with embodiments of the invention described hererin. No claim is made to such a method of promotion. What is meant by "promoting" is promoting, advertising, or marketing. Examples of promoting include, but are not limited to, written, visual, or-verbal statements made on the product or in stores, magazines, newspaper, radio, television, internet, and the like. Examples of such statements include, but are not limited to: reduces the appearance of wrinkles and/or fine lines, lifts the skin, firms the skin, reduces the appearance of the age of the skin, provides younger looking skin, and similar statements.

EXAMPLES

The following is a description of examples of measurements of rates of propagation of mechanical energy (resonance running time), determination of skin anisotropy parameters, and related methods for evaluating and promoting skin treatments. Other methods within the scope of the present invention can practiced in an analogous manner by a person of ordinary skill in the art.

Example 1

Resonance running time versus probe angle for 239 human subjects was determined using the Reviscometer® RVM 600. The subjects were of varying ethnicity and skin type, from very light Caucasian (Types I and II) to African Americans (Type VI). The volunteers were divided in 5 groups according to their age: 0-2 years old (mean age of $1.8 \pm$ standard deviation of 1.1); 14-20 years old ($17 \pm 4.2$), 24-40 years old ($32.5 \pm 10.6$), 55-60 y old ($57.5 \pm 3.5$); and 65-75 years old ($70 \pm 7$). Reviscometer readings were taken on the upper inner arm at about 15cm from the elbow for a range of probe angles that spanned 100°. The measurements were taken in 3° increments, and 0° was (arbitrarily) assigned to the angle that gave the lowest RRT reading. An example of a plot of resonance running time ("RRT") versus angle for one individual is shown in Figure 4. RRT is expressed in "RRT Units," each of which is about 1/10,000 seconds.

After taking these measurements, a 6 term-Gaussian function was fitted to the measured RRT curve as a function the angle using the computer program IDL® (from RSI, Research System Inc.., Boulder, CO). Two anisotropy parameters were calculated: (1) the ratio of the maximum resonance running time ($RRT_{max}$) divided by the minimum resonance running time ($RRT_{min}$), referred to hereinafter as anisotropy ("A"); and (2) and the full width at half maximum of the Gaussian distribution, referred to as the Langer's Line Width ("LW"). These anisotropy parameters are illustrated in the example in Figure 4.

Example 2

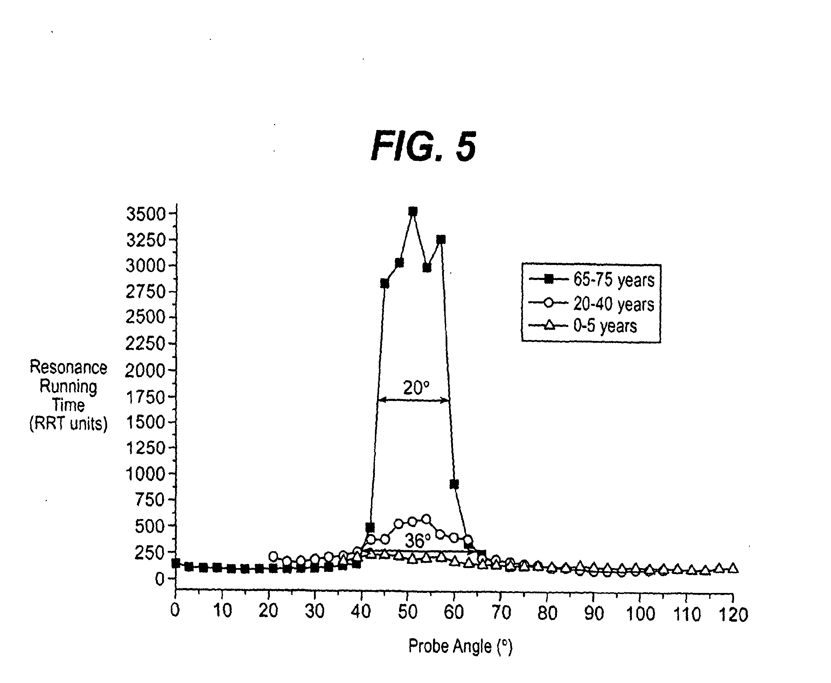

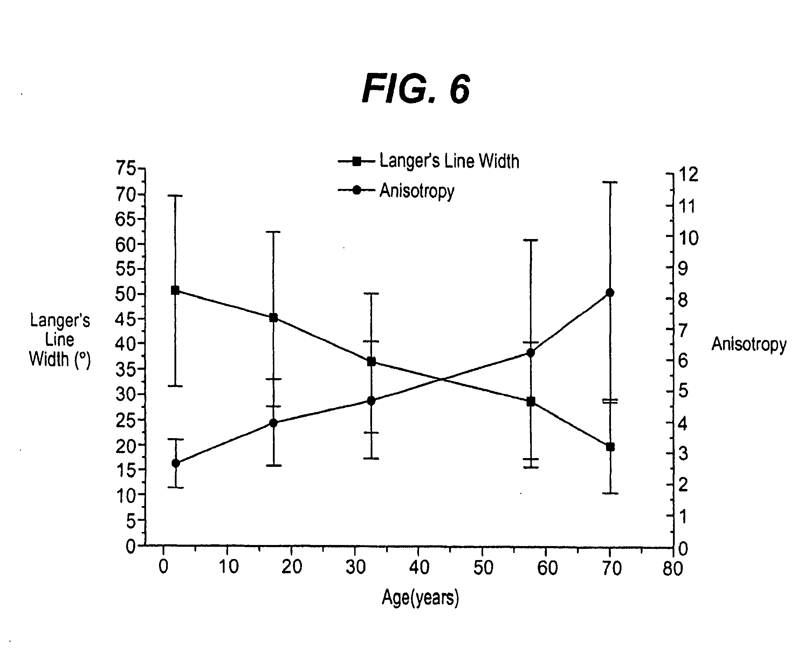

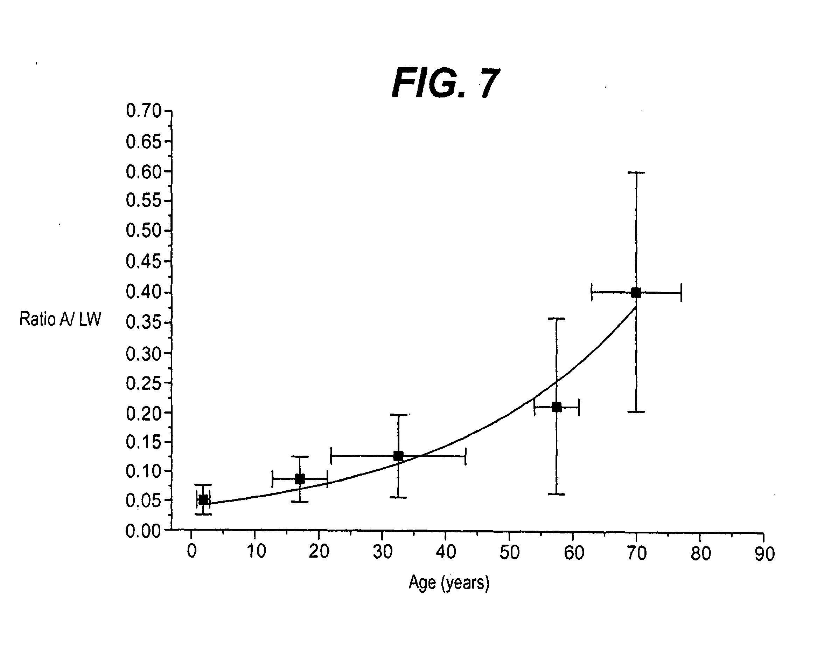

The subjects of Example 1 were categorized by age group. A representative example of RRT as a function of angle for each of three age groups is shown in Figure 5. It should be noted that the plot of RRT versus angle is substantially different for subjects of different ages. In particular, the magnitude of A increased significantly with age. Figure 6 shows the means (shown as circles/squares in the Figure) and standard deviation (shown as bars in the Figure) of both A and LW for all 5 age groups. It is clear from Figure 6 that LW falls with increasing age of the subjects. As shown in Figure 7, a model was developed that correlated a third skin anisotropy parameter, the ratio of A/LW, to chronological age of the subject. The model was developed using analysis of variance (ANOVA), and had a high degree of statistical significance (p<0.001). The model predicts:

$$\frac{A}{LW} = 0.041 \cdot e^{\frac{age}{26.02}}$$

This fitted expression may be used to calculate a test subject's age based upon his A/LW ratio. Solving for age, one obtains:

$$\text{Age} = 26.01 \cdot \left( \ln\left(\frac{A}{LW}\right) + \ln(0.041) \right)$$

Example 3

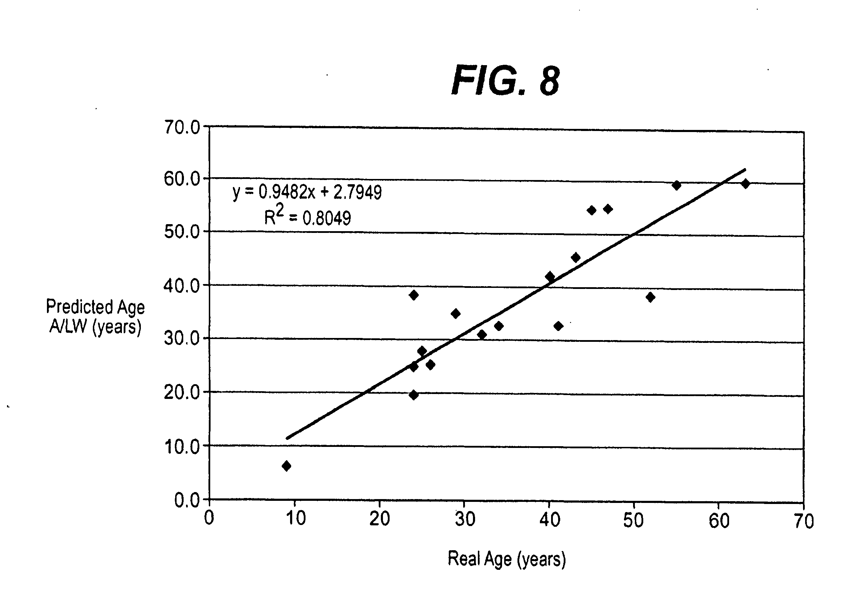

The model from Example 2 was used to calculate various subjects' ages based on their particular ratio, A/ LW, calculated from the Reviscometer measurements. This was done on 18 subjects with real age varying from 9 up to 63 years old. The actual age in years and the predicted age using the above expression are illustrated in Figure 8. There is a good correlation between the actual age of the subjects and their predicted age (correlation coefficient, $R^2 > 0.8$).

Example 4

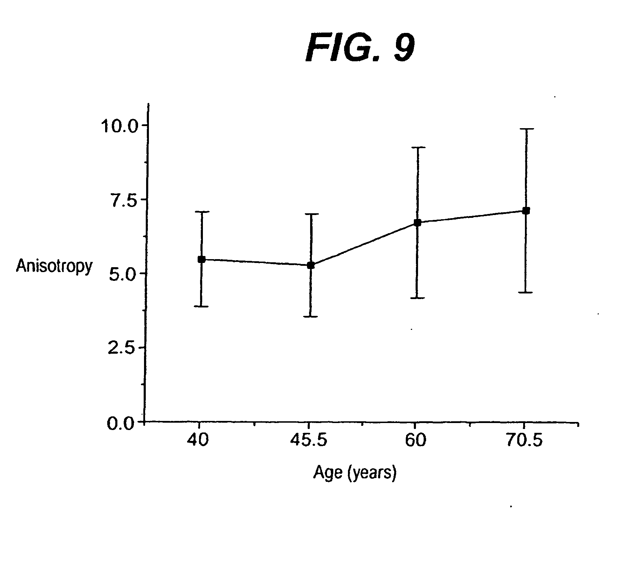

Skin anisotropy of 84 human subjects was determined using the Reviscometer® RVM 600. The subjects were Caucasian women ranging in age from 40 to 72 years. Reviscometer readings were taken on the neck area half way from the bottom of the ear area to the collarbone, in an interval ranging from 0° up to 100° in 3° increments, where the initial 0° is an arbitrary angle that gives us the lowest RRT reading. Gaussian curves were fitted to the data and the values of the A and LW were calculated. After statistical analysis using ANOVA (general linear model), no statistical significance was found for LW as a function of age (p=0.62) but there was statistical significance for the A (p=0.026). A plot of A versus age category is shown below in Figure 9.

Example 5

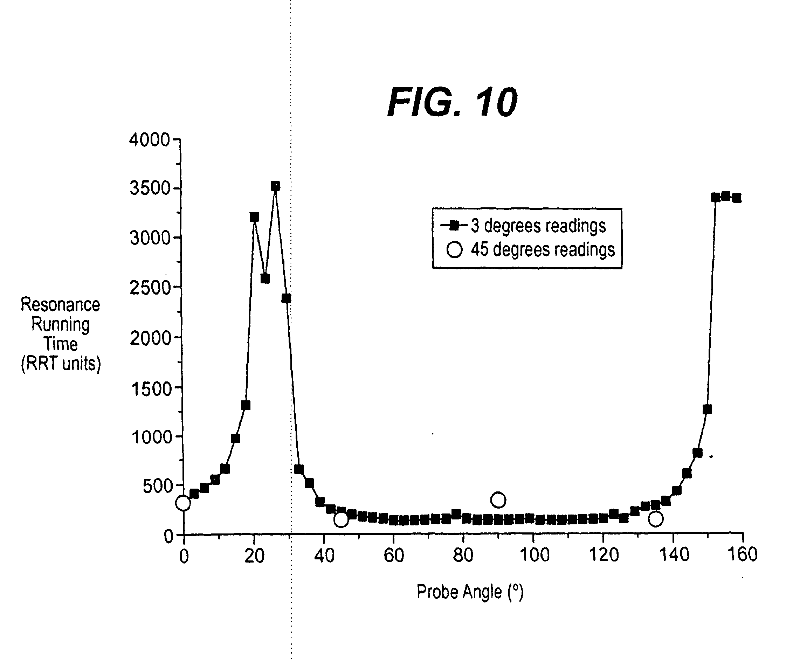

Figure 10 is a plot of RRT vs. angle for an individual subject of the study specified in Example 1. It can be seen that if measurements were only taken at 45° intervals (proposed by the manufacturer), as opposed to more frequently such as every 3°, important information is lost, thereby dramatically reducing the ability to discriminate anisotropy using the device.

Example 6

Skin anisotropy of 6 human subjects was determined using the Reviscometer® RVM 600. Reviscometer readings were taken on the upper inner arm at about 15cm from the elbow in an interval ranging from 0° up to 128° in 3° increments. For each subject, one of six topical skin treatments were separately applied to different expanses of skin (using a dose of 2 $\mu$l/cm$^2$). Reviscometer readings were then taken 35 minutes after treatment. The topical treatments that were tested included 0%, 0.5%, 1%, 2%, and 3% dimethyl amino ethanol (DMAE), by weight, formulated in an identical cosmetic base (additional DMAE was compensated for by using less water).

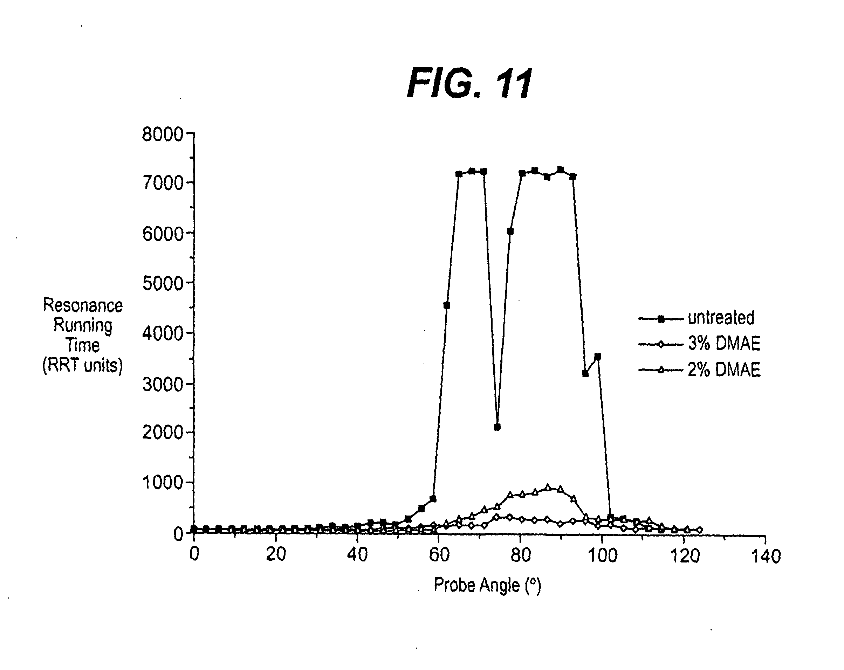

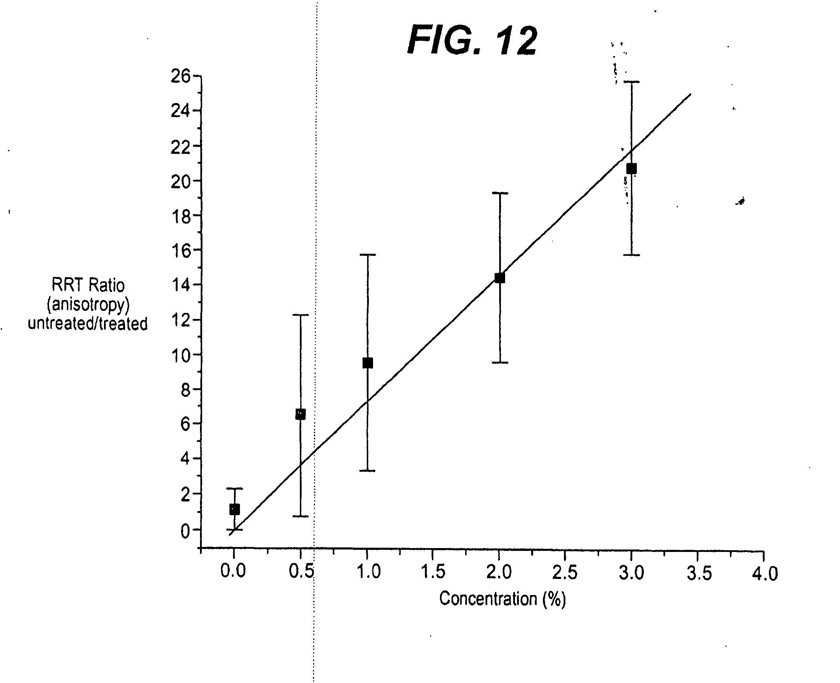

Figure 11 shows the Reviscometer readings (Resonance Time) as a function of the probe angle on skin for pre-treated (i.e., prior to treatment with the DMAE composition), treated with 2% DMAE, and treated with 3% DMAE. As shown in Figure 11, the application of DMAE on skin decreases the values of the resonance response, as a result of tightening of the skin. The efficacy of the skin treatment was determined, as shown in Figure 12. The degree to which a skin anisotropy parameter is enhanced from treatment of the skin with a benefit agent is plotted versus dose of the benefit agent. The degree of enhancement of anisotropy or "RRT ratio" (RRTR) is:

$$\text{RRT ratio} = \frac{(RRT\max° - RRT\min°)\text{ untreated}}{(RRT\max° - RRT\min°)\text{ treated}}$$

where *RRT* is the resonance running time (Reviscometer readings) taken at maximum and minimum values.

Definition of the RRT allowed the calculation of a dose response relationship between RRTR and the concentration of DMAE. Applying the same procedure for eight volunteers using the 5 samples determined a dose-response function of the concentration. Figure 12 shows the dose-response relationship for the concentration of DMAE, by weight, indicating a 20-fold decrease in RRT for 3% DMAE treatment. This post-treatment RRT could also be compared to a standard "age" category as well to associate the treated skin with a particular age grouping.

Embodiments of the invention described herein are advantageous in that clear and subtle differences in skin elasticity may be determined. For example, by taking measurements with small angle separations, one is likely to include measurements that are closely aligned with the particular Langer lines of the expanse of skin that is being measured. As such, this may permit measuring enormous differences in properties (e.g., a 3 fold, 6 fold or even greater difference in rate of propagation of mechanical energy). Because the various embodiments of the inventive method provide excellent resolution, subtle differences may be measured, thereby allowing the inventive measurement method to (1) be used to promote skin care treatments, including topical treatments; and/or (2) to make assessments regarding chronological age of the skin based on its elastic properties.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1.   A method of determining skin anisotropy of a subject, said method comprising measuring rates of propagation of mechanical energy between a mechanical energy generator (5) and a mechanical energy detector (7) along a plurality of directions (19, 21) of an expanse of skin (17), **characterised in that** each of said directions (19,21) are from about 0° to about 10° in separation relative to at least one other of said directions (19,21) and at least two of said directions (19,21) are from about 30° to about 180° in separation relative to each other.

2.   A method of claim 1, wherein at least two of said directions (19,21) are at least about 90° in separation relative to each other.

3.   A method of claim 1 or claim 2, wherein each of said directions (19,21) are from about 0° to about 5° in separation relative to least one other of said directions (19,21).

4.   A method of claim 1, 2 or 3, wherein a distance between said mechanical energy generator (5) and said mechanical energy detector (7) is from about 0.5mm and about 5mm.

5.   A method of claim 1, 2, 3 or 4 wherein said mechanical energy generator (5) and said mechanical energy detector (7) are transducers.

6.   A method of any preceding claim wherein the expanse of skin (17) has a location selected from a group consisting of the upper inner arm, the jaw, the upper inner thigh, the abdomen, and the neck.

7.   A method of any of claims 1 to 6 wherein the expanse of skin (17) is located on the upper inner arm.

8.   A method of any preceding claim wherein said mechanical energy comprises an elastic shear wave.

9.   A method of any preceding claim wherein said mechanical energy has a frequency from about 0.5 kHz to about 30 kHz.

10. A method of any preceding claim, said method further comprising determining a skin anisotropy parameter based on said measured rates and comparing said skin anisotropy parameter to a standard skin anisotropy parameter that is associated with skin age.

11. A method of determining the efficacy of a skin treatment said comprise the steps of:

(i) determining a first skin anisotropy measurement of a subject by the method of any preceding claim
(ii) administering a non-surgical cosmetic treatment to said expanse of skin; and
(iii) determining a second skin anisotropy measurement of the subject by the method of any preceding claim and
(iv) comparing said first and said second skin anisotropy measurements.

12. A method of claim 11 wherein said treatment comprises applying a topical composition to the expanse of skin (17).

13. A method of claim 11 wherein said treatment comprises applying a device to the expanse of skin (17).

**Patentansprüche**

1. Verfahren zum Bestimmen der Hautanisotropie einer Person, wobei das Verfahren das Messen der Fortpftanzungsgeschwindigkeit mechanischer Energie zwischen einem Generator (5) für mechanische Energie und einem Detektor (7) für mechanische Energie entlang einer Vielzahl von Richtungen (19, 21) einer Fläche der Haut (17) aufweist, **dadurch gekennzeichnet, dass** jede der Richtungen (19, 21) um einen Bogen von ungefähr 0° bis ungefähr 10° relativ zu wenigstens einer anderen der Richtungen (19, 21) getrennt ist, und dass wenigstens zwei der Richtungen (19, 21) ungefähr 30° bis ungefähr 180° in Bezug aufeinander getrennt sind.

2. Verfahren nach Anspruch 1, bei dem wenigstens zwei der Richtungen (19, 21) wenigstens ungefähr 90° in Bezug aufeinander getrennt sind.

3. Verfahren nach Anspruch 1 oder Anspruch 2, bei dem jede der Richtungen (19, 21) ungefähr 0° bis ungefähr 5° in Bezug auf wenigstens eine andere der Richtungen (19, 21) getrennt ist.

4. Verfahren nach Anspruch 1, 2 oder 3, bei dem ein Abstand zwischen dem Generator (5) für mechanische Energie und dem Detektor (7) für mechanische Energie ungefähr 0.5 mm bis ungefähr 5 mm beträgt.

5. Verfahren nach Anspruch 1, 2, 3 oder 4, hei dem der Generator (5) für mechanische Energie und der Detektor (7) für mechanische Energie Transducer sind.

6. Verfahren nach einem der vorangehenden Ansprüche, bei dem die Fläche der Haut (17) an einem Ort liegt, der aus einer Gruppe bestehend aus dem inneren Oberarm, dem Kiefer, dem inneren Oberschenkel, dem Unterleib und dem Hals ausgewählt ist.

7. Verfahren nach einem der Ansprüche 1 bis 6, bei der sich die Fläche der Haut (17) auf dem inneren Oberarm befindet.

8. Verfahren nach irgendeinem Anspruch, der Anspruch 8 vorausgeht, bei dem die mechanische Energie eine elastische Scherwelle aufweist.

9. Verfahren nach einem der vorangehenden Ansprüche, bei dem die mechanische Energie eine Frequenz von ungefähr 0.5 kHz bis ungefähr 30 kHz hat.

10. Verfahren nach einem der vorangehenden Ansprüche, wobei das Verfahren weiter das Bestimmen eines Hautanisotropieparameters basierend auf den gemessenen Geschwindigkeiten und das Vergleichen des Hautanisotropieparameters mit einem Standard-Haulanisolropieparameter, der mit dem Hautalter verknüpft ist, aufweist.

11. Verfahren zum Bestimmten der Wirksamkeit einer Hautbehandlung, wobei das Verfahren die Schritte aufweist:

(i) Bestimmen einer ersten Hautanisotropiemessung bei einer Person durch das Verfahren nach einem der vorangehenden Ansprüche;
(ii) Anwenden einer nicht chirurgischen kosmetischen Behandlung auf die Fläche der Haut; und

(iii) Bestimmen einer zweiten Hautanisotropiemessung bei der Person durch das Verfahren nach einem der vorangehenden Ansprüche; und
(iv) Vergleichen der ersten und der zweiten Hautanisotropicmcssung.

12. Verfahren nach Anspruch 11, bei dem die Behandlung das Anwenden einer zeitgemäßen Zusammensetzung auf die Fläche der Haut (17) aufwcist.

13. Verfahren nach Anspruch 11, bei dem die Behandlung das Anwenden eines Gerätes auf die Fläche der Haut (17) aufweist.

**Revendications**

1. Procédé pour déterminer l'anisotropie de la peau d'un sujet, ledit procédé comprenant la mesure de vitesses de propagation d'énergie mécanique entre un générateur d'énergie mécanique (5) et un détecteur d'énergie mécanique (7) le long d'une pluralité de directions (19, 21) de l'étendue de la peau (17), **caractérisé en ce que** chacune desdites directions (19, 21) est séparée relativement d'environ 0° à environ 10° par rapport à au moins une autre desdites directions (19, 21) et au moins deux desdites directions (19, 21) sont séparées relativement d'environ 30° à environ 180° l'une par rapport à l'autre.

2. Procédé selon la revendication 1, dans lequel au moins deux desdites directions (19, 21) sont au moins séparées relativement, d'environ 90° l'une par rapport à l'autre.

3. Procédé Selon la revendication 1 ou la revendication 2, dans lequel chacune desdites directions (19, 21) est séparée relativement d'environ 0° à environ 5° par rapport à au moins une autre desdites directions (19, 21).

4. Procédé selon la revendication 1, 2 ou 3, dans lequel une distance entre ledit générateur d'énergie mécanique (5) et ledit détecteur d'énergie mécanique (7) est d'environ 0,5 mm à environ 5 mm.

5. Procédé selon la revendication 1, 2, 3 ou 4, dans lequel ledit générateur d'énergie mécanique (5) et ledit détecteur d'énergie mécanique (7) sont des transducteurs.

6. Procédé selon l'une quelconque des revendications précédentes, dans lequel l'étendue de la peau (17) a un emplacement choisi dans le groupe constitué par l'intérieur du haut du bras, la mâchoire, l'intérieur du haut de la cuisse, l'abdomen et le cou.

7. Procédé selon l'une quelconque des revendications 1 à 6, dans lequel l'étendue de la peau (17) est située sur l'intérieur du haut du bras.

8. Procède selon l'une quelconque des revendications précédentes, dans lequel ladite énergie mécanique comprend une onde de cisaillement. élastique.

9. Procédé selon l'une quelconque des revendications précédentes, dans lequel ladite énergie mécanique a une fréquence d'environ 0,b kHz à environ 30 KHz.

10. Procédé selon l'une quelconque des revendications précédentes, ledit procède comprenant en outre la détermination d'un paramètre d'anisotropie de la peau basé sur lesdites vitesses mesurées et la comparaison dudit paramètre d'anisotropie de la peau à un paramètre d'anisotropie de la peau type qui est associé à l'âge de la peau.

11. Procède pour déterminer l'efficacité d'un traitement de la peau, ledit procédé comprenant les étapes consistant à :

(i) déterminer une première mesure d'anisotropie de la peau d'un sujet par le procédé selon l'une quelconque des revendications précédentes;
(ii) administrer un traitement cosmétique non chirurgical à ladite étendue de la peau ; et
(iii) déterminer une seconde mesure d'anisotropie de la peau du sujet par le procédé selon l'une quelconque des revendications précédentes ; et
(iv) comparer ladite première et ladite seconde mesure d'anisotropie de la peau.

**12.** Procédé selon la revendication 11, dans lequel ledit traitement comprend l'application d'une composition topique à l'étendue de la peau (17).

**13.** Procédé selon la revendication 11, dans lequel ledit traitement comprend l'application d' un dispositif à l'étendue de la peau (17).

# FIG. 1

# FIG. 2A

# FIG. 2B

**FIG. 3**

# FIG. 4

**FIG. 5**

EP 1 634 532 B1

## FIG. 6

**FIG. 7**

FIG. 8

y = 0.9482x + 2.7949
$R^2$ = 0.8049

Predicted Age
A/LW (years)

Real Age (years)

## FIG. 9

# FIG. 10

EP 1 634 532 B1

## FIG. 11

EP 1 634 532 B1

*FIG. 12*

EP 1 634 532 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6168572 B
- US 20020029924 A

- US 5115808 A

**Non-patent literature cited in the description**

- **Vexler et al.** *The Journal of Investigative Dermatology, Inc.,* 1999, vol. 115 (5), 732-739